# EUROPEAN PATENT APPLICATION

(11) **EP 4 137 155 A1**
(43) Date of publication of application: **22.02.2023**
(21) Application number: 21788967.4
(22) Date of filing: 15.04.2021
(51) Int. Cl.: A61K 39/395, C07K 16/28, A61P 35/00

(54) **PREPARATION SPECIFICALLY BOUND WITH CD137 AND USE THEREOF**

(30) Priority: 17.04.2020 CN 202010305482
(71) Applicant: Dingfu Biotarget Co., Ltd, Suzhou, Jiangsu 215125 (CN)
(72) Inventor: ZHANG, Xin, Suzhou, Jiangsu 215125 (CN); DONG, Yanrong, Suzhou, Jiangsu 215125 (CN); PENG, Jianjian, Suzhou, Jiangsu 215125 (CN); FU, Kai, Suzhou, Jiangsu 215125 (CN); WANG, Li, Suzhou, Jiangsu 215125 (CN)
(74) Representative: Böhm, Brigitte
(86) International application number: PCT/CN2021/087582
(87) International publication number: WO 2021/209017

(57) **Abstract**

Provided by the present application is a preparation, which comprises an antibody specifically bound with CD137 or an antigen-binding fragment thereof, wherein the CD137 antibody comprises a light chain variable region VL and a heavy chain variable region VH, wherein the VL comprises an amino acid sequence shown in any one of SEQ ID NO:1-11, and the VH comprises an amino acid sequence shown in any one of SEQ ID NO:12-22. Also provided by the present application are a kit comprising the preparation, and a use of the preparation or the kit in the preparation of a drug, the drug being used for the treatment of a cancer.

## Description

### FIELD OF THE INVENTION

The present application relates to the field of biomedicine, and in particular to a preparation specifically binding to CD137 and use thereof.

### BACKGROUND OF THE INVENTION

Cancer is a disease that seriously threatens human health. In recent years, immunotherapy, as a new therapy, has shown great potential in oncotherapy.

CD137 (also known as 4-1BB or TNFRSF9) is a type I transmembrane protein and a member of the tumor necrosis factor receptor superfamily. CD137L is a type II transmembrane protein and a member of the tumor necrosis factor receptor superfamily. Studies have indicated that CD137L is mainly expressed on activated APCs, such as dendritic cells (DCs), macrophages and B cells (Pollok, KE et al., 1994, Eur.J.Immunol. 24: 367-374); while CD137 may be induced to express after T cells receive antigen-specific signals (Kwon, B.S. et al., 1989, PNAS 86: 1963-67). The effects of CD137 on T cells have been well demonstrated. In the presence of a certain amount of anti-CD3 antibody, activation of CD137 signaling may induce the proliferation of T cells and the synthesis of cytokines (mainly IFN-γ), and inhibit the apoptosis of activated T cells, thereby prolonging the life of T cells.

However, currently available anti-CD137 antibodies have low stability and limited tumor inhibition; as a result, it is urgent to develop new anti-CD137 drugs for new drug development.

### SUMMARY OF THE INVENTION

In one aspect, the present application provides a preparation comprising an antibody or an antigen-binding fragment thereof that specifically binds to CD137, wherein the anti-CD137 antibody comprises a light chain variable region VL and a heavy chain variable region VH, wherein the VL comprises an amino acid sequence as set forth in any one of SEQ ID NOs: 1-11; and the VH comprises an amino acid sequence as set forth in any one of SEQ ID NOs: 12-22.

In some embodiments, in the preparation,
1) the VL comprises an amino acid sequence as set forth in SEQ ID NO: 1, and the VH comprises an amino acid sequence as set forth in SEQ ID NO: 12;
2) the VL comprises an amino acid sequence as set forth in SEQ ID NO: 3, and the VH comprises an amino acid sequence as set forth in SEQ ID NO: 14;
3) the VL comprises an amino acid sequence as set forth in SEQ ID NO: 5, and the VH comprises an amino acid sequence as set forth in SEQ ID NO: 16;
4) the VL comprises an amino acid sequence as set forth in SEQ ID NO: 7, and the VH comprises an amino acid sequence as set forth in SEQ ID NO: 18;
5) the VL comprises an amino acid sequence as set forth in SEQ ID NO: 8, and the VH comprises an amino acid sequence as set forth in SEQ ID NO: 19;
6) the VL comprises an amino acid sequence as set forth in SEQ ID NO: 9, and the VH comprises an amino acid sequence as set forth in SEQ ID NO: 20;
7) the VL comprises an amino acid sequence as set forth in SEQ ID NO: 10, and the VH comprises an amino acid sequence as set forth in SEQ ID NO: 21;
8) the VL comprises an amino acid sequence as set forth in SEQ ID NO: 11, and the VH comprises an amino acid sequence as set forth in SEQ ID NO: 22.

In some embodiments, the preparation comprises a buffer salt selected from one or more of the group consisting of: phosphate, citrate and acetate.

In some embodiments, the preparation comprises a buffer salt comprising disodium hydrogen phosphate, sodium dihydrogen phosphate and/or sodium citrate.

In some embodiments, the preparation comprises a buffer salt at a concentration of 5-50 mM.

In some embodiments, the preparation comprises a stabilizer selected from one or more of the group consisting of sucrose, trehalose, mannitol, glycine, arginine hydrochloride, sodium citrate, histidine, sodium acetate and sodium chloride.

In some embodiments, the preparation comprises a stabilizer comprising sucrose.

In some embodiments, the preparation comprises a stabilizer comprising sucrose and sodium citrate.

In some embodiments, the preparation comprises a stabilizer at a concentration of 1%-10% (wt).

In some embodiments, the preparation comprises a surfactant selected from one or more of the group consisting of: polysorbate 20 and polysorbate 80.

In some embodiments, the preparation comprises a surfactant at a concentration of 0.01%-0.2% (wt).

In some embodiments, the preparation has a pH of 6.5-7.5.

In some embodiments, the preparation comprises:
sucrose, polysorbate 80, disodium hydrogen phosphate, sodium dihydrogen phosphate, sodium citrate and water.

In another aspect, the present application provides a kit comprising the preparation and a container for containing the preparation.

In another aspect, the present application provides use of the preparation or the kit in the preparation of a drug for treating cancer.

In some embodiments, the cancer is selected from the group consisting of: melanoma, prostate cancer, colorectal cancer, Merkel cell carcinoma, pancreatic cancer, non-Hodgkin's lymphoma, squamous cell carcinoma and breast cancer.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application may be better understood by referring to the exemplary embodiments and the accompanying drawings described in detail below. A brief description of the drawings is as follows:
FIGs. 1A-1B show that the preparation described herein has a significant killing effect on tumors *in vivo.*
FIGs. 2A-2C show that the preparation described herein has a significant killing effect on tumors *in vivo.*
FIG. 3 shows that the preparation described herein significantly promotes the secretion of cytokines by T cells.

### DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

The present application is further described as follows: in the present invention, unless otherwise specified, the scientific and technical terms used herein have the meanings commonly understood by those skilled in the art. Moreover, as used herein, terms and laboratory operating steps in relation to the protein and nucleic acid chemistry, molecular biology, cell and tissue culture, microbiology, and immunology are all terms and routine steps widely used in the corresponding fields. At the same time, for a better understanding of the present invention, definitions and explanations of related terms are provided below.

As used herein, the term "preparation" generally refers to a pharmaceutical composition comprising an active ingredient and a pharmaceutically acceptable carrier or excipient. For example, in the present application, the preparation may comprise an antibody or an antigen-binding fragment thereof that specifically binds to CD137, where the CD137 antibody comprises a light chain variable region VL and a heavy chain variable region VH, where the VL comprises an amino acid sequence as set forth in any one of SEQ ID NOs: 1-11, and the VH contains an amino acid sequence as set forth in any one of SEQ ID NOs: 12-22. In addition, in some embodiments, the preparation in the present application may also contain at least one of a buffer salt, a stabilizer and a surfactant.

In the present application, the term "antibody" generally refers to a polypeptide molecule that can specifically recognize and/or neutralize a specific antigen. For example, the antibody may comprise an immunoglobulin consisting of at least two heavy (H) chains and two light (L) chains that are interconnected by disulfide bonds, and may include any molecule comprising an antigen-binding portion thereof. The term "antibody" comprises monoclonal antibodies, antibody fragments or antibody derivatives, including but not limited to human antibodies, humanized antibodies, chimeric antibodies, single domain antibodies (for example, dAb), single-chain antibodies (for example, scFv), and antibody fragments (for example, Fab, Fab' and (Fab) 2 fragments) that bind to the antigen. The term "antibody" also comprises all recombinant forms of antibodies, such as antibodies expressed in prokaryotic cells, and any antigen-binding antibody fragments and derivatives thereof described herein. Each heavy chain may consist of a heavy-chain variable region (VH) and a heavy-chain constant region. Each light chain may consist of a light-chain variable region (VL) and a light-chain constant region. The VH and VL regions may be further distinguished as hypervariable regions that are called complementarity determining regions (CDRs), which are interspersed in more conserved regions that are called framework regions (FRs). Each VH or VL may consist of three CDRs and four FR regions, which may be arranged in the following order from an amino terminus to a carboxy terminus: FR1, CDR1, FR2, CDR2, FR3, CDR3, and FR4. The heavy-chain and light-chain variable regions comprise binding domains that interact with antigens. The constant region of the antibody can mediate the binding of the immunoglobulin to a host tissue or factor, where the host tissue or factor comprises various cells of an immune system (e.g., effector cells) and a first component (Clq) of the classical complement system.

In the present application, the term "antigen-binding fragment" generally refers to one or more fragments in the antibody that fulfill the function of specifically binding to an antigen. The antigen-binding function of an antibody can be achieved by a full-length fragment of the antibody. The antigen-binding function of an antibody may also be achieved by the following fragments: (1) an Fab fragment, i.e., a monovalent fragment consisting of VL, VH, CL and CH domains; (2) an F(ab')2 fragment, a bivalent fragment comprising two Fab fragments linked through disulfide bonds at a hinge region; (3) an Fd fragment consisting of VH and CH domains; (4) an Fv fragment consisting of VL and VH domains of a single arm of an antibody; (5) a dAb fragment consisting of VH domains (Ward et al., (1989) Nature 341: 544-546); (6) an isolated complementarity determining region (CDR) and (7) a combination of two or more isolated CDRs that may optionally be linked by linkers. In addition, the above fragments may further comprise a monovalent single-chain Fv molecule (scFv) formed by pairing VL and VH (see Bird et al. (1988) Science 242: 423-426; and Huston et al. (1988) Proc.Natl.Acad.Sci. 85: 5879-5883). The "antigen-binding portion" may further comprise an immunoglobulin fusion protein, where the fusion protein comprises a binding domain selected from the following: (1) a binding domain polypeptide fused to an immunoglobulin hinge region polypeptide; (2) an immunoglobulin heavy chain CH2 constant region fused to the hinge region; and (3) an immunoglobulin heavy chain CH3 constant region fused to the CH2 constant region. The antibody or the antigen-binding fragment thereof in the present application is selected from the group consisting of Fab, scFv, Fab', F(ab)2, F(ab')2 and dAb.

In the present application, the term "CD137 protein", also referred to as 4-1BB or TNFRS9, generally refers to a transmembrane protein of the tumor necrosis factor receptor superfamily (TNFRS), which is an activation-induced costimulatory molecule and an important regulator of immune response. Studies have shown that a CD137 agonistic monoclonal antibody increases the expression of the costimulatory molecule in many models, and significantly improves the response of cytolytic T lymphocytes, thus playing an anti-tumor effect. The anti-tumor effect of CD137-targeted therapy may be verified through the study on the anti-tumor efficacy of the agonistic anti-mouse CD137 monoclonal antibody in mice. CD137 has become a potent activator of immune cells, and an important candidate antigen for treating various diseases (see Vinay, Dass S., and Byoung S.Kwon."4-1BB (CD137), an inducible costimulatory receptor, as a specific target for cancer therapy." BMB reports 47.3 (2014): 122).

In the present application, the term "antibody Fc domain" generally refers to a Y-shaped base region in the structure of an antibody, also referred to as a fragment crystallizable region (Fc region). In antibody isotypes of IgG, IgA and IgD, the Fc region consists of two identical protein fragments, which come from second and third constant domains of two heavy chains of the antibody; Fc regions in IgM and IgE comprise three heavy chain constant domains in each polypeptide chain. The Fc region in IgG has highly conservative N-glycosylation sites. In the present application, the Fc domain may comprise an amino acid sequence as set forth in SEQ ID: 34.

In the present application, the term "specific binding" generally refers to a measurable and reproducible interaction, such as the binding between a target and an antibody, and the existence of the target may be determined in a heterogeneous population of molecules (including biomolecules). For example, an antibody that specifically binds to a target (which may be an epitope) is an antibody that binds to the target with greater affinity, avidity, easiness, and/or duration than it binds to other targets. In one embodiment, the extent to which an antibody binds to an unrelated target is about 10% less than the extent to which the antibody binds to a target, as measured, for example, by radioimmunoassay (RIA).

In the present application, the term "kit" generally refers to a collection of various components, where the components may or may not be packaged together. For example, in the present application, the kit may consist of the preparation and a container for containing the preparation. The components in the kit may be contained in separate vials (i.e., a kit with separate parts), or provided within a single vial. In addition, the kit may include instructions which may be available in a printed or electronic user's manual. For example, the manual may contain instructions for interpreting the results obtained when using the kit of the present application.

In the present application, the term "cancer" generally refers to or describes a physiological condition of mammals, with the typical feature consisting in the disorder of cell proliferation or survival. In the present application, hyperproliferative diseases referred to as cancers include, but are not limited to, a solid tumor, lymphoma and leukemia.

In the present application, the term "subject" generally refers to a human or non-human animal, including but not limited to a cat, a dog, a horse, a pig, a cow, a goat, a rabbit, a mouse, a rat, or a monkey.

In the present application, the term "administration" generally refers to a method of administering a certain dose of preparation and drug to a subject (for example, a patient). The administration may be carried out by any suitable means. Various dosing schedules are encompassed herein, including but not limited to a single administration or multiple administrations over various time points.

In the present application, homology of an amino acid or a nucleotide sequence may be used interchangeably with the term "identity". The term "identity" generally refers to the percentage of identical residues that are paired. The "percent sequence identity" is calculated by comparing the two optimally aligned sequences over a specific region; determining the number of positions in the two sequences at which the identical base or amino acid occurs to obtain the number of matched positions; dividing the number of such positions by the total number of positions in the compared section, and multiplying the resulting quotient by 100. As described above, a program for determining homology (or identity) is used to compare aligned sequences on the basis of amino acid-to-amino acid, and the program can set different levels of stringency for the alignment (e.g., identical amino acids, conservative amino acid substitutions, etc.). As the term is used herein, two amino acids discussed are considered "conservative substitutions" for each other if each belongs to the same chemical class (i.e., acidic, non-polar/hydrophobic, uncharged polar and basic). As a non-limiting example, two different amino acids belonging to a non-polar amino acid will be considered "conservative substitutions" for each other, even if the two amino acids are not identical, and will not be considered "conservative substitutions" for each other when they are non-polar amino acids on the one hand, and basic amino acids on the other hand. Amino acids are divided into four major groups: acidic, non-polar, uncharged polar and basic (Alberts, Johnson, Lewis, Raff, Roberts and Walter, MolecularBiology of the Cell, 4th edition, (2002) column 3.1). This grouping may be used in the context of the present invention for the purpose of determining whether a specific amino acid is a conservative substitution for another amino acid discussed. The above major groups may be further subdivided into, for example, small non-polar and large non-polar amino acids, large aromatic amino acids, etc. The term "conservative amino acid substitution" also refers to any amino acid substitution for a given amino acid residue, wherein the substituted residue is chemically so similar to the given residue that there is no substantial reduction in the results of polypeptide function (e.g., binding).

In the present application, the term "containing" generally refers to the inclusion of explicitly specified features, but not excluding other elements.

In the present application, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### Preparation and kit

### Antibody or antigen-binding fragment thereof

In one aspect, the present application provides a preparation comprising an antibody or an antigen-binding fragment thereof that specifically binds to CD137, wherein the anti-CD137 antibody comprises a light chain variable region VL and a heavy chain variable region VH, wherein the VL comprises an amino acid sequence as set forth in any one of SEQ ID NOs: 1-11; and the VH comprises an amino acid sequence as set forth in any one of SEQ ID NOs: 12-22.

In the present application, in the antibody or the antigen-binding fragment thereof that specifically binds to CD137, the VL may comprise an amino acid sequence as set forth in SEQ ID NO: 1, and the VH may comprise an amino acid sequence as set forth in SEQ ID NO: 12.

In the present application, in the antibody or the antigen-binding fragment thereof that specifically binds to CD137, the VL may comprise an amino acid sequence as set forth in SEQ ID NO: 2, and the VH may comprise an amino acid sequence as set forth in SEQ ID NO: 13.

In the present application, in the antibody or the antigen-binding fragment thereof that specifically binds to CD137, the VL may comprise an amino acid sequence as set forth in SEQ ID NO: 3, and the VH may comprise an amino acid sequence as set forth in SEQ ID NO: 14;

In the present application, in the antibody or the antigen-binding fragment thereof that specifically binds to CD137, the VL may comprise an amino acid sequence as set forth in SEQ ID NO: 4, and the VH may comprise an amino acid sequence as set forth in SEQ ID NO: 15.

In the present application, in the antibody or the antigen-binding fragment thereof that specifically binds to CD137, the VL may comprise an amino acid sequence as set forth in SEQ ID NO: 5, and the VH may comprise an amino acid sequence as set forth in SEQ ID NO: 16;

In the present application, in the antibody or the antigen-binding fragment thereof that specifically binds to CD137, the VL may comprise an amino acid sequence as set forth in SEQ ID NO: 6, and the VH may comprise an amino acid sequence as set forth in SEQ ID NO: 17.

In the present application, in the antibody or the antigen-binding fragment thereof that specifically binds to CD137, the VL may comprise an amino acid sequence as set forth in SEQ ID NO: 7, and the VH may comprise an amino acid sequence as set forth in SEQ ID NO: 18;

In the present application, in the antibody or the antigen-binding fragment thereof that specifically binds to CD137, the VL may comprise an amino acid sequence as set forth in SEQ ID NO: 8, and the VH may comprise an amino acid sequence as set forth in SEQ ID NO: 19;

In the present application, in the antibody or the antigen-binding fragment thereof that specifically binds to CD137, the VL may comprise an amino acid sequence as set forth in SEQ ID NO: 9, and the VH may comprise an amino acid sequence as set forth in SEQ ID NO: 20;

In the present application, in the antibody or the antigen-binding fragment thereof that specifically binds to CD137, the VL may comprise an amino acid sequence as set forth in SEQ ID NO: 10, and the VH may comprise an amino acid sequence as set forth in SEQ ID NO: 21;

In the present application, in the antibody or the antigen-binding fragment thereof that specifically binds to CD137, the VL may comprise an amino acid sequence as set forth in SEQ ID NO: 11, and the VH may comprise an amino acid sequence as set forth in SEQ ID NO: 22.

In the present application, the antibody or the antigen-binding fragment thereof that specifically binds to CD137 may further comprise an Fc domain. For example, the Fc region of the antibody may comprise an Fc region of an IgG. For example, the Fc domain of the antibody may comprise a constant region amino acid sequence selected from the group consisting of immunoglobulins: IgG1, IgG2, IgG3 and IgG4. In some embodiments, the Fc domain may comprise an amino acid sequence as set forth in SEQ ID NO: 34.

In the present application, the Fc domain of the antibody or the antigen-binding fragment thereof that specifically binds to CD137 may be located at the C-terminus of the antibody or the antigen-binding fragment thereof.

In the present application, a sequence of the Fc of the antibody or the antigen-binding fragment thereof that specifically binds to CD137 may comprise an amino acid sequence as set forth in SEQ ID NO: 34 or a variant thereof.

In the present application, the antibody or the antigen-binding fragment thereof that specifically binds to CD137 may be a homodimeric protein consisting of 2 polypeptide chains, wherein each polypeptide chain comprises the light chain variable region VL, the heavy chain variable region VH and the Fc domain, wherein the Fc domain is located at the C-terminus of the VL and the VH.

In the present application, the light chain variable region and the heavy chain variable region in the antibody or the antigen-binding fragment thereof that specifically binds to CD137 may further comprise a linker peptide 1, wherein the linker peptide 1 may comprise an amino acid sequence as set forth in SEQ ID NO: 35 or a variant thereof. For example, the linker peptide 1 may comprise (G4S)3.

In the present application, the light chain variable region and the Fc in the antibody or the antigen-binding fragment thereof that specifically binds to CD137 may further comprise a linker peptide 2, wherein the linker peptide 2 may comprise an amino acid sequence as set forth in SEQ ID NO: 36 or a variant thereof. For example, the linker peptide 2 may comprise G4S.

In the present application, in the antibody or the antigen-binding fragment thereof that specifically binds to CD137, the polypeptide chain may comprise an amino acid sequence as set forth in any one of ID NO: NO.23 -33.

For example, the antibody or the antigen-binding fragment thereof that specifically binds CD137 may comprise antibodies C2-4A-Fc, C2-4B-Fc, C2-4C-Fc, C2-5A-Fc, C2-5B-Fc, C2-5C-Fc, C2-6A-Fc, C2-6B-Fc, C2-7A-Fc, C2-7B-Fc or C2-7BN78K-Fc.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 1 or a variant thereof; and the heavy chain thereof may comprise a heavy chain variable region, wherein an amino acid sequence of the heavy chain variable region may comprise an amino acid sequence as set forth in SEQ ID NO: 12 or a variant thereof. For example, the antibody or the antigen-binding fragment thereof may comprise antibody C2-4A-Fc or the antibody described herein having identical light chain variable region and heavy chain variable region thereto.

For example, the antibody described herein may be C2-4A-Fc. In the antibody C2-4A-Fc, an amino acid sequence of VL is as set forth in SEQ ID NO: 1, an amino acid sequence of VH is as set forth in SEQ ID NO: 12, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 23.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 2 or a variant thereof; and the heavy chain thereof may comprise a heavy chain variable region, wherein an amino acid sequence of the heavy chain variable region may comprise an amino acid sequence as set forth in SEQ ID NO: 13 or a variant thereof. For example, the antibody or the antigen-binding fragment thereof may comprise antibody C2-4B-Fc or the antibody described herein having identical light chain variable region and heavy chain variable region thereto.

For example, the antibody described herein may be C2-4B-Fc. In the antibody C2-4B-Fc, an amino acid sequence of VL is as set forth in SEQ ID NO: 2, an amino acid sequence of VH is as set forth in SEQ ID NO: 13, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 24.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 3 or a variant thereof; and the heavy chain thereof may comprise a heavy chain variable region, wherein an amino acid sequence of the heavy chain variable region may comprise an amino acid sequence as set forth in SEQ ID NO: 14 or a variant thereof. For example, the antibody or the antigen-binding fragment thereof may comprise antibody C2-4C-Fc or the antibody described herein having identical light chain variable region and heavy chain variable region thereto.

For example, the antibody described herein may be C2-4C-Fc. In the antibody C2-4C-Fc, an amino acid sequence of VL is as set forth in SEQ ID NO: 3, an amino acid sequence of VH is as set forth in SEQ ID NO: 14, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 25.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 4 or a variant thereof; and the heavy chain thereof may comprise a heavy chain variable region, wherein an amino acid sequence of the heavy chain variable region may comprise an amino acid sequence as set forth in SEQ ID NO: 15 or a variant thereof. For example, the antibody or the antigen-binding fragment thereof may comprise antibody C2-5A-Fc or the antibody described herein having identical light chain variable region and heavy chain variable region thereto.

For example, the antibody described herein may be C2-5A-Fc. In the antibody C2-5A-Fc, an amino acid sequence of VL is as set forth in SEQ ID NO: 4, an amino acid sequence of VH is as set forth in SEQ ID NO: 15, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 26.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 5 or a variant thereof; and the heavy chain thereof may comprise a heavy chain variable region, wherein an amino acid sequence of the heavy chain variable region may comprise an amino acid sequence as set forth in SEQ ID NO: 16 or a variant thereof. For example, the antibody or the antigen-binding fragment thereof may comprise antibody C2-5B-Fc or the antibody described herein having identical light chain variable region and heavy chain variable region thereto.

For example, the antibody described herein may be C2-5B-Fc. In the antibody C2-5B-Fc, an amino acid sequence of VL is as set forth in SEQ ID NO: 5, an amino acid sequence of VH is as set forth in SEQ ID NO: 16, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 27.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 6 or a variant thereof; and the heavy chain thereof may comprise a heavy chain variable region, wherein an amino acid sequence of the heavy chain variable region may comprise an amino acid sequence as set forth in SEQ ID NO: 17 or a variant thereof. For example, the antibody or the antigen-binding fragment thereof may comprise antibody C2-5C-Fc or the antibody described herein having identical light chain variable region and heavy chain variable region thereto.

For example, the antibody described herein may be C2-5C-Fc. In the antibody C2-5C-Fc, an amino acid sequence of VL is as set forth in SEQ ID NO: 6, an amino acid sequence of VH is as set forth in SEQ ID NO: 17, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 28.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 7 or a variant thereof; and the heavy chain thereof may comprise a heavy chain variable region, wherein an amino acid sequence of the heavy chain variable region may comprise an amino acid sequence as set forth in SEQ ID NO: 18 or a variant thereof. For example, the antibody or the antigen-binding fragment thereof may comprise antibody C2-6A-Fc or the antibody described herein having identical light chain variable region and heavy chain variable region thereto.

For example, the antibody described herein may be C2-6A-Fc. In the antibody C2-6A-Fc, an amino acid sequence of VL is as set forth in SEQ ID NO: 7, an amino acid sequence of VH is as set forth in SEQ ID NO: 18, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 29.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 8 or a variant thereof; and the heavy chain thereof may comprise a heavy chain variable region, wherein an amino acid sequence of the heavy chain variable region may comprise an amino acid sequence as set forth in SEQ ID NO: 19 or a variant thereof. For example, the antibody or the antigen-binding fragment thereof may comprise antibody C2-6B-Fc or the antibody described herein having identical light chain variable region and heavy chain variable region thereto.

For example, the antibody described herein may be C2-6B-Fc. In the antibody C2-6B-Fc, an amino acid sequence of VL is as set forth in SEQ ID NO: 8, an amino acid sequence of VH is as set forth in SEQ ID NO: 19, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 30.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 9 or a variant thereof; and the heavy chain thereof may comprise a heavy chain variable region, wherein an amino acid sequence of the heavy chain variable region may comprise an amino acid sequence as set forth in SEQ ID NO: 20 or a variant thereof. For example, the antibody or the antigen-binding fragment thereof may comprise antibody C2-7A-Fc or the antibody described herein having identical light chain variable region and heavy chain variable region thereto.

For example, the antibody described herein may be C2-7A-Fc. In the antibody C2-7A-Fc, an amino acid sequence of VL is as set forth in SEQ ID NO: 9, an amino acid sequence of VH is as set forth in SEQ ID NO: 20, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 31.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 10 or a variant thereof; and the heavy chain thereof may comprise a heavy chain variable region, wherein an amino acid sequence of the heavy chain variable region may comprise an amino acid sequence as set forth in SEQ ID NO: 21 or a variant thereof. For example, the antibody or the antigen-binding fragment thereof may comprise antibody C2-7B-Fc or the antibody described herein having identical light chain variable region and heavy chain variable region thereto.

For example, the antibody described herein may be C2-7B-Fc. In the antibody C2-7B-Fc, an amino acid sequence of VL is as set forth in SEQ ID NO: 10, an amino acid sequence of VH is as set forth in SEQ ID NO: 21, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 32.

In some embodiments, the light chain of the antibody or the antigen-binding fragment thereof that specifically binds to CD137 described herein may comprise a light chain variable region, wherein an amino acid sequence of the light chain variable region may comprise an amino acid sequence as set forth in SEQ ID NO: 11 or a variant thereof; and the heavy chain thereof may comprise a heavy chain variable region, wherein an amino acid sequence of the heavy chain variable region may comprise an amino acid sequence as set forth in SEQ ID NO: 22 or a variant thereof. For example, the antibody or the antigen-binding fragment thereof may comprise antibody C2-7BN78K-Fc or the antibody described herein having identical light chain variable region and heavy chain variable region thereto.

For example, the antibody described herein may be C2-7BN78K-Fc. In the antibody C2-7BN78K-Fc, an amino acid sequence of VL is as set forth in SEQ ID NO: 11, an amino acid sequence of VH is as set forth in SEQ ID NO: 22, and a sequence of Fc is as set forth in SEQ ID NO: 34; a linker peptide between VL and VH is (G4S)3 having a sequence as set forth in SEQ ID NO: 35; a linker peptide between VL and Fc is G4S having a sequence as set forth in SEQ ID NO: 36; and a complete sequence of each polypeptide chain is as set forth in SEQ ID NO: 33.

In the present application, a variant of the amino acid sequence may be an amino acid sequence that has substantially the same function (for example, is capable of specifically binding to CD137 protein) as the amino acid sequence and that has at least about 85% (for example, at least about 85%, about 90%, about 91%, about 92%, about 93%, about 94%, about 95%, about 96%, about 97%, about 98%, about 99%, or higher) sequence identity thereto. In some embodiments, a variant of the amino acid sequence is an amino acid sequence that has substantially the same function (for example, is capable of specifically binding to CD137 protein) as the amino acid sequence and that comprises, based thereon, one or more (for example, 1-2, 1-3, 1-4, 1-5, 1-6, 1-7, 1-8, 1-9, 1-10, or more) additions, deletions or substitutions of amino acids.

In some embodiments, the antibody may be at a concentration of 5-50 mg/mL. For example, in some cases, the antibody may be at a concentration of 5-50mg/mL, 5-45mg/mL, 5-40mg/mL, 5-35mg/mL, 5-30mg/mL, 5-25mg/mL, 5-20mg/mL, 6-50mg/mL, 6-45mg/mL, 6-40mg/mL, 6-35mg/mL, 6-30mg/mL, 6-25mg/mL, 6-20mg/mL, 7-50mg/mL, 7-45mg/mL, 7-40mg/mL, 7-35mg/mL, 7-30mg/mL, 7-25mg/mL, 7-20mg/mL, 8-50mg/mL, 8-45mg/mL, 8-40mg/mL, 8-35mg/mL, 8-30mg/mL, 8-25mg/mL, 8-20mg/mL, 9-50mg/mL, 9-45mg/mL, 9-40mg/mL, 9-35mg/mL, 9-30mg/mL, 9-25mg/mL, 9-20mg/mL, 10-50mg/mL, 10-45mg/mL, 10-40mg/mL, 10-35mg/mL, 10-30mg/mL, 10-25mg/mL, 10-20mg/mL, 10-50mg/mL, 15-45mg/mL, 15-40mg/mL, 15-35mg/mL, 15-30mg/mL, 15-25mg/mL or 15-20mg/mL.

### Buffer salt, stabilizer, surfactant and kit

In the present application, the preparation may further comprise, in addition to the antibody or the antigen-binding fragment thereof that specifically binds to CD137, a buffer salt, wherein the buffer salt may be selected from one or more of the group consisting of: phosphate, citrate and acetate.

In some embodiments, the buffer salt may comprise disodium hydrogen phosphate, sodium dihydrogen phosphate and/or sodium citrate. For example, in some cases, the buffer salt may comprise disodium hydrogen phosphate and sodium dihydrogen phosphate. For another example, in some cases, the buffer salt may comprise disodium hydrogen phosphate, sodium dihydrogen phosphate and sodium citrate.

In some embodiments, the buffer salt may be at a concentration of 5-50 mM. For example, in some cases, the buffer salt may be at a concentration of 5-10 mM, 5-20 mM, 5-30 mM, 5-40 mM, 5-50 mM, 10-20 mM, 10-30 mM, 10-40 mM, 10-50 mM, 15-20 mM, 15-30 mM, 15-40 mM, 15-50 mM, 20-30 mM, 20-40 mM, 20-50 mM, 25-30 mM, 25-40 mM, 25-50 mM, 30-40 mM, 30-50 mM or 40-50 mM. For example, in some cases, the buffer salt may be at a concentration of 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, 30 mM, 31 mM, 32 mM, 33 mM, 34 mM or 35 mM.

In the present application, the buffer salt may comprise disodium hydrogen phosphate and sodium dihydrogen phosphate. For example, in the preparation described herein, the sum of the concentration of the disodium hydrogen phosphate and the sodium dihydrogen phosphate may be 10-20 mM, 10-30 mM, 10-40 mM, 10-50 mM, 15-20 mM, 15-30 mM, 15-40 mM, 15-50 mM, 20-30 mM, 20-40 mM, 20-50 mM, 25-30 mM, 25-40 mM, 25-50 mM, 30-40 mM, 30-50 mM or 40-50 mM. For another example, in the preparation described herein, the sum of the concentration of the disodium hydrogen phosphate and the sodium dihydrogen phosphate may be 1%-2% (wt), 1%-3% (wt), 1%-4% (wt), 1%-5% (wt), 1%-6% (wt), 1%-7% (wt), 1%-8% (wt), 1%-9% (wt), 1%-10% (wt), 2%-3% (wt), 2%-4% (wt), 2%-5% (wt), 2%-6% (wt), 3%-4% (wt), 3%-5% (wt), 3%-6% (wt), 3.5%-4% (wt), 3.5%-5% (wt) or 3%-6% (wt).

In the present application, the buffer salt may comprise sodium citrate. For example, in the preparation described herein, the sucrose may be at a concentration of 0.1%-6% (wt), 0.1%-6.5% (wt), 0.1%-7% (wt), 0.1%-7.5% (wt), 0.1%-8% (wt), 0.1%-8.5% (wt), 0.1%-9% (wt), 0.1%-9.5% (wt), 0.1%-10% (wt), 0.2%-6% (wt), 0.2%-6.5% (wt), 0.2%-7% (wt), 0.2%-7.5% (wt), 0.2%-8% (wt), 0.2%-8.5% (wt), 0.2%-9% (wt), 0.2%-9.5% (wt), 0.2%-10% (wt), 0.3%-6% (wt), 0.3%-6.5% (wt), 0.3%-7% (wt), 0.3%-7.5% (wt), 0.3%-8% (wt), 0.3%-8.5% (wt), 0.3%-9% (wt), 0.3%-9.5% (wt) or 0.3%-10% (wt).

In the present application, the preparation may further comprise, in addition to the antibody or the antigen-binding fragment thereof that specifically binds to CD137, a stabilizer, wherein the stabilizer may be selected from one or more of the group consisting of: sucrose, trehalose, mannitol, glycine, arginine hydrochloride, sodium citrate, histidine, sodium acetate and sodium chloride. For example, in some embodiments, the stabilizer may comprise sucrose. For another example, in some embodiments, the stabilizer may comprise sucrose and sodium citrate.

In some embodiments, the stabilizer may be at a concentration of 1%-10% (wt). For example, in some cases, the stabilizer may be at a concentration of 1%-2% (wt), 1%-3% (wt), 1%-4% (wt), 1%-5% (wt), 1%-6% (wt), 1%-7% (wt), 1%-8% (wt), 1%-9% (wt), 1%-10% (wt), 2%-3% (wt), 2%-4% (wt), 2%-5% (wt), 2%-6% (wt), 2%-7% (wt), 2%-8% (wt), 2%-9% (wt), 2%-10% (wt), 3%-4% (wt), 3%-5% (wt), 3%-6% (wt), 3%-7% (wt), 3%-8% (wt), 3%-9% (wt), 3%-10% (wt), 4%-5% (wt), 4%-6% (wt), 4%-7% (wt), 4%-8% (wt), 4%-9% (wt), 4%-10% (wt), 5%-6% (wt), 5%-7% (wt), 5%-8% (wt), 5%-9% (wt), 5%-10% (wt), 6%-7% (wt), 6%-8% (wt), 6%-9% (wt), 6%-10% (wt), 6.5%-7% (wt), 6.5%-8% (wt), 6.5%-9% (wt), 6.5%-10% (wt), 6.8%-7% (wt), 6.8%-8% (wt), 6.8%-9% (wt) or 6.8%-10% (wt).

In some embodiments, the stabilizer may be at a concentration of 1%-10% (wt). For example, in some cases, the stabilizer may be at a concentration of 5 mM to 250 mM. For example, in some cases, the buffer salt may be at a concentration of 5-250 mM, 5-240 mM, 5-230 mM, 5-220 mM, 5-210 mM, 5-200 mM, 5-190 mM, 5-180 mM, 5-170 mM, 5-160 mM, 5-150 mM, 10-250 mM, 10-240 mM, 10-230 mM, 10-220 mM, 10-210 mM, 10-200 mM, 10-190 mM, 10-180 mM, 10-170 mM, 10-160 mM or 10-150 mM.

In the present application, the stabilizer may comprise sucrose. For example, in some cases, the sucrose may be at a concentration of 5-250 mM, 5-240 mM, 5-230 mM, 5-220 mM, 5-210 mM, 5-200 mM, 5-190 mM, 10-250 mM, 10-240 mM, 10-230 mM, 10-220 mM, 10-210 mM, 10-200 mM, 10-190 mM, 50-250 mM, 50-240 mM, 50-230 mM, 50-220 mM, 50-210 mM, 50-200 mM or 50-190 mM.

In the present application, the stabilizer may comprise sodium citrate which may be, for example, sodium citrate dihydrate. For example, in some cases, the sodium poncirate may be at a concentration of 5-250 mM, 5-240 mM, 5-230 mM, 5-220 mM, 5-210 mM, 5-200 mM, 5-190 mM, 5-180 mM, 5-170 mM, 5-160 mM, 5-150 mM, 10-250 mM, 10-240 mM, 10-230 mM, 10-220 mM, 10-210 mM, 10-200 mM, 10-190 mM, 10-180 mM, 10-170 mM, 10-160 mM or 10-150 mM.

In the present application, the stabilizer may comprise sucrose. For example, in the preparation described herein, the sucrose may be at a concentration of 1%-6% (wt), 1%-6.5% (wt), 1%-7% (wt), 1%-7.5% (wt), 1%-8% (wt), 1%-8.5% (wt), 1%-9% (wt), 1%-9.5% (wt), 1%-10% (wt), 2%-6% (wt), 2%-6.5% (wt), 2%-7% (wt), 2%-7.5% (wt), 2%-8% (wt), 2%-8.5% (wt), 2%-9% (wt), 2%-9.5% (wt), 2%-10% (wt), 3%-6% (wt), 3%-6.5% (wt), 3%-7% (wt), 3%-7.5% (wt), 3%-8% (wt), 3%-8.5% (wt), 3%-9% (wt), 3%-9.5% (wt) or 3%-10% (wt).

In the present application, the stabilizer may comprise sodium citrate. For example, in the preparation described herein, the sucrose may be at a concentration of 0.1%-6% (wt), 0.1%-6.5% (wt), 0.1%-7% (wt), 0.1%-7.5% (wt), 0.1%-8% (wt), 0.1%-8.5% (wt), 0.1%-9% (wt), 0.1%-9.5% (wt), 0.1%-10% (wt), 0.2%-6% (wt), 0.2%-6.5% (wt), 0.2%-7% (wt), 0.2%-7.5% (wt), 0.2%-8% (wt), 0.2%-8.5% (wt), 0.2%-9% (wt), 0.2%-9.5% (wt), 0.2%-10% (wt), 0.3%-6% (wt), 0.3%-6.5% (wt), 0.3%-7% (wt), 0.3%-7.5% (wt), 0.3%-8% (wt), 0.3%-8.5% (wt), 0.3%-9% (wt), 0.3%-9.5% (wt) or 0.3%-10% (wt).

In the present application, the preparation may further comprise, in addition to the antibody or the antigen-binding fragment thereof that specifically binds to CD137, a surfactant, wherein the surfactant may be selected from one or more of the group consisting of: polysorbate 20 and polysorbate 80. For example, the surfactant may comprise polysorbate 20 (i.e., PS20). For another example, the surfactant may comprise polysorbate 80 (i.e., PS80). For another example, the surfactant may comprise polysorbate 20 and polysorbate 80.

In some embodiments, the surfactant may be at a concentration of 0.01%-0.2% (wt). For example, in some cases, the surfactant may be at a concentration of 0.01%-0.2% (wt), 0.02%-0.2% (wt), 0.03%-0.2% (wt), 0.04%-0.2% (wt), 0.05%-0.2% (wt), 0.06%-0.2% (wt), 0.07%-0.2% (wt), 0.08%-0.2% (wt), 0.09%-0.2% (wt), 0.1%-0.2% (wt) or 0.15%-0.2% (wt).

In the present application, the surfactant may comprise polysorbate 80. For example, in the preparation described herein, the polysorbate 80 may be at a concentration of 0.01%-0.2% (wt), 0.02%-0.2% (wt), 0.03%-0.2% (wt), 0.04%-0.2% (wt), 0.05%-0.2% (wt), 0.06%-0.2% (wt), 0.07%-0.2% (wt), 0.08%-0.2% (wt), 0.09%-0.2% (wt), 0.1%-0.2% (wt) or 0.15%-0.2% (wt).

In the present application, the preparation may have a pH of 6.5-7.5. For example, in some cases, the preparation may have a pH of 6.5-6.6, 6.5-6.7, 6.5-6.8, 6.5-6.9, 6.5-7.0, 6.5-7.1, 6.5-7.2, 6.5-7.3, 6.5-7.4, 6.5-7.5, 6.6-6.7, 6.6-6.8, 6.6-6.9, 6.6-7.0, 6.6-7.1, 6.6-7.2, 6.6-7.3, 6.6-7.4, 6.6-7.5, 6.7-6.8, 6.7-6.9, 6.7-7.0, 6.7-7.1, 6.7-7.2, 6.7-7.3, 6.7-7.4, 6.7-7.5, 6.8-6.9, 6.8-7.0, 6.8-7.1, 6.8-7.2, 6.8-7.3, 6.8-7.4 or 6.8-7.5. For example, in some cases, the preparation may have a pH of 6.6-7.2.

In the present application, the preparation may further comprise, in addition to the antibody or the antigen-binding fragment thereof that specifically binds to CD137, sucrose, polysorbate 80 (i.e., PS80), disodium hydrogen phosphate, sodium dihydrogen phosphate, sodium citrate and water.

In the present application, the preparation may consist of an antibody or an antigen-binding fragment thereof that specifically binds to CD137, sodium dihydrogen phosphate, disodium hydrogen phosphate, sucrose, sodium citrate, histidine, polysorbate 80 and water.

In the present application, the preparation may consist of an antibody or an antigen-binding fragment thereof that specifically binds CD137, about 20 mM stabilizer (sodium dihydrogen phosphate and disodium hydrogen phosphate), about 4.8%-6.2% (wt) sucrose, about 10 mM sodium citrate, about 50 mM histidine, about 0.05% (wt) polysorbate 80, and water.

In another aspect, the present application provides a kit comprising the preparation and a container for containing the preparation. In the present application, the kit generally refers to a collection of various components, where the components may or may not be packaged together. For example, in the present application, the kit may consist of the preparation and a container for containing the preparation. The components in the kit may be contained in separate vials (i.e., a kit with separate parts), or provided within a single vial. In addition, the kit may include instructions which may be available in a printed or electronic user's manual. For example, the manual may contain instructions for interpreting the results obtained when using the kit of the present application.

### Use

In another aspect, the present application provides use of the preparation or the kit in the preparation of a drug for treating cancer.

In another aspect, the present application provides a method for treating cancer, wherein the method comprises administering the preparation or the kit described herein to a subject in need thereof.

In another aspect, the preparation or the kit described herein may be used to treat cancer.

In the present application, the preparation or the kit may promote the secretion of cytokines by T cells (for example, the T cells may secrete IFN-g).

In the present application, the preparation may be formulated into an injection, so that the preparation described herein may be used for intravenous injection or subcutaneous injection. In some embodiments, the preparation described herein may also be formulated for oral administration, intravenous administration, intramuscular administration, *in situ* administration at a tumor site, inhalation, rectal administration, vaginal administration, transdermal administration or administration via a subcutaneous depot.

In the present application, the administration method may be intratumoral injection, for example, injection of the preparation described herein into the interior of a tumor. In some embodiments, the administration method may also be oral administration, intravenous administration, intramuscular administration, *in situ* administration at a tumor site, inhalation, rectal administration, vaginal administration, transdermal administration or administration via a subcutaneous depot.

In some embodiments, the dose level of the preparation described herein administered to a subject may vary depending on: the molecule delivered, the administration route, and the size (body weight, body surface or organ size) and/or physical condition (age and general health) of the patient.

In the present application, the cancer may be selected from the group consisting of: melanoma, prostate cancer, colorectal cancer, Merkel cell carcinoma, pancreatic cancer, non-Hodgkin's lymphoma, squamous ce
11 carcinoma and breast cancer.

### Examples

The following examples are given merely to illustrate the preparation, the use, etc. according to the present application, and are not intended to limit the scope of the present application, nor are they intended to represent that the tests described below are all and the only tests performed. Efforts have been made to ensure accuracy of values (e.g., amounts, temperature, etc.) used, however, some experimental errors and deviations should be considered. Unless otherwise indicated, parts are parts by weight, molecular weight is weight-average molecular weight, temperature is in degrees Celsius, and pressure is at or near normal atmospheric pressure. Standard abbreviations may be used, for example, bp, base pair; kb, kilobase pair; pL, picoliter; s or sec, seconds; min, minutes; h or hr, hour; aa, amino acid; nt, nucleotide; iv, intravenous injection; i.m., intramuscularly; i.p., intraperitoneally; s.c., subcutaneously; etc.

### Example 1. Preparation of Anti-CD137 Antibodies

### 1.1 Structures of anti-CD137 antibodies

Amino acid sequences of the anti-CD137 antibodies of the present application are shown in Table 1.

**Table 1. Amino acid sequences of anti-CD137 antibodies**

| **No.** | **Name of antibody** | **VL (SEQ ID No:)** | **VH (SEQ ID No:)** | **Complete sequence of each polypeptide chain (SEQ ID NO:)** |
|---|---|---|---|---|
| 1 | C2-4A-Fc | 1 | 12 | 23 |
| 2 | C2-4B-Fc | 2 | 13 | 24 |
| 3 | C2-4C-Fc | 3 | 14 | 25 |
| 4 | C2-5A-Fc | 4 | 15 | 26 |
| 5 | C2-5B-Fc | 5 | 16 | 27 |
| 6 | C2-5C-Fc | 6 | 17 | 28 |
| 7 | C2-6A-Fc | 7 | 18 | 29 |
| 8 | C2-6B-Fc | 8 | 19 | 30 |
| 9 | C2-7A-Fc | 9 | 20 | 31 |
| 10 | C2-7B-Fc | 10 | 21 | 32 |
| 11 | C2-7BN78K-Fc | 11 | 22 | 33 |

### 1.2 Expression and purification of anti-CD137 antibodies

The amino acid sequences in Table 1 were used to design the corresponding coding DNA sequences by utilizing DNAworks online tool (http://helixweb.nih.gov/dnaworks/); the obtained DNA sequences were digested with Hind III and EcoR I of Fermentas Biotechnology company and then cloned to a commercial vector pcDNA4, and the accuracy of the constructed plasmids was verified by sequencing to obtain recombinant plasmid DNAs, namely: pcDNA4-C2-4A-Fc, pcDNA4-C2-4B-Fc, pcDNA4-C2-4C-Fc, pcDNA4-C2-5A-Fc, pcDNA4-C2-5B-Fc, pcDNA4-C2-5C-Fc, pcDNA4-C2-6A-Fc, pcDNA4-C2-6B-Fc, pcDNA4-C2-7A-Fc, pcDNA4-C2-7B-Fc and pcDNA4-C2-7BN78K-Fc. Plasmid extraction was performed according to standard procedures for molecular cloning, and the extracted plasmids were transiently expressed in HEK 293 cells and purified by Protein A column to obtain C2-4A-Fc, C2-4B-Fc, C2-4C-Fc, C2-5A-Fc, C2-5B-Fc, C2-5C-Fc, C2-6A-Fc, C2-6B-Fc, C2-7A-Fc, C2-7B-Fc and C2-7BN78K-Fc which were used in the following examples. The obtained protein samples were preliminarily detected by SDS-PAGE, and the band of interest could be clearly seen.

### Example 2. Preparation of Preparations and Performance Detection Therefor

Preparations 1-14 (i.e., the preparations described herein) were prepared by mixing the anti-CD137 antibody prepared in Example 1 with one or more of a buffer salt, a stabilizer and a surfactant and adjusting the pH of the mixture; the types and contents of the components are shown in Tables 2-1, 2-2 and 2-3, wherein "/" indicates no component contained.

**Table 2-1. Components of preparation and content thereof**

| **Preparation** | **Antibody** | | **pH** | **Buffer salt** | |
|---|---|---|---|---|---|
| | **Designation** | **Concentration (mg/mL)** | | **Type** | **Concentration (mM)** |
| 1 | C2-7BN78K-Fc | 10 | 6.5 | Citric acid and sodium citrate | 20 |
| 2 | C2-7BN78K-Fc | 10 | 6.5 | Sodium dihydrogen phosphate and disodium hydrogen phosphate | 20 |
| 3 | C2-7BN78K-Fc | 10 | 7.0 | Sodium dihydrogen phosphate and disodium hydrogen phosphate | 20 |
| 4 | C2-7BN78K-Fc | 20 | 7.0 | Sodium dihydrogen phosphate and disodium hydrogen phosphate | 20 |
| 5 | C2-7BN78K-Fc | 20 | 7.0 | Sodium dihydrogen phosphate and disodium hydrogen phosphate | 20 |
| 6 | C2-7BN78K-Fc | 20 | 7.0 | Sodium dihydrogen phosphate and disodium hydrogen phosphate | 20 |
| 7 | C2-7BN78K-Fc | 20 | 7.0 | Sodium dihydrogen phosphate and disodium hydrogen phosphate | 20 |
| 8 | C2-7BN78K-Fc | 20 | 7.0 | Sodium dihydrogen phosphate and disodium hydrogen phosphate | 20 |
| 9 | C2-7BN78K-Fc | 20 | 7.0 | Sodium dihydrogen phosphate and disodium hydrogen phosphate | 20 |
| 10 | C2-7BN78K-Fc | 20 | 7.0 | Sodium dihydrogen phosphate and disodium hydrogen phosphate | 20 |
| 11 | C2-7BN78K-Fc | 20 | 7.0 | Sodium dihydrogen phosphate and disodium hydrogen phosphate | 20 |
| 12 | C2-7BN78K-Fc | 20 | 7.0 | Sodium dihydrogen phosphate and disodium hydrogen phosphate | 20 |
| 13 | C2-7BN78K-Fc | 10 | 6.6 | Sodium dihydrogen phosphate and disodium hydrogen phosphate | 20 |
| 14 | C2-7BN78K-Fc | 15 | 7.2 | Sodium dihydrogen phosphate and disodium hydrogen phosphate | 20 |

**Table 2-2. Components of preparation and content thereof**

| **Preparation** | **Stabilizer** | | | | | |
|---|---|---|---|---|---|---|
| | **Type** | **Content (%, wt)** | **Type** | **Concentration (mM)** | **Type** | **Concentration (mM)** |
| 1 | / | / | / | / | / | / |
| 2 | / | / | / | / | / | / |
| 3 | / | / | / | / | / | / |
| 4 | Sucrose | 7.5 | / | / | / | / |
| 5 | Trehalose | 8 | / | / | / | / |
| 6 | Sucrose | 7.5 | / | / | / | / |
| 7 | Trehalose | 8 | / | / | / | / |
| 8 | Sucrose | 4.8 | Sodium citrate | 5 | / | / |
| 9 | Sucrose | 4.8 | Sodium citrate | 10 | / | / |
| 10 | Sucrose | 4.8 | Sodium citrate | 20 | / | / |
| 11 | Sucrose | 4.8 | Sodium citrate | 10 | Histidine | 50 |
| 12 | Sucrose | 4.8 | Sodium citrate | 10 | Histidine | 100 |
| 13 | Sucrose | 4.8 | Sodium citrate | 10 | Histidine | 50 |
| 14 | Sucrose | 6.2 | Sodium citrate | 10 | Histidine | 50 |

**Table 2-3. Components of preparation and content thereof**

| **Preparation** | **Surfactant** | |
|---|---|---|
| | **Type** | **Content (%, wt)** |
| 1 | / | / |
| 2 | / | / |
| 3 | / | / |
| 4 | / | / |
| 5 | / | / |
| 6 | PS80 | 0.05 |
| 7 | PS20 | 0.05 |
| 8 | PS80 | 0.05 |
| 9 | PS80 | 0.05 |
| 10 | PS80 | 0.05 |
| 11 | PS80 | 0.05 |
| 12 | PS80 | 0.05 |
| 13 | PS80 | 0.05 |
| 14 | PS80 | 0.05 |

The performance of preparations 1-14 was detected, and the main test contents and methods are briefly described as follows:

### (1) Appearance

The appearance of the samples was visually detected. The sample bottles were wiped clean and placed in a clarity detector (purchased from Tianda Tianfa Technology Co., Ltd) under 1000lux of light to observe the color, clarity and visible impurities of the samples.

### (2) pH value

The pH value of the samples were detected by adopting a pH meter (available from Mettler Toledo). The pH meter was calibrated with pH calibration solutions at pH 4.01, pH 7.00 and pH 9.21 to ensure slopes above 95%.

### (3) Protein concentration

The protein concentration was detected by adopting an ultraviolet spectrophotometer (purchased from Agilent). The samples were diluted with ultrapure water, so that an absorbance value thereof was in a range of 0.3-0.7 Abs. The absorbance of the samples at 280 nm wavelength was detected, the sample concentration was calculated, an extinction coefficient was 1.749, and protein concentration = absorbance value / extinction coefficient × dilution factor.

### (4) Size-exclusion high-performance liquid chromatography (SEC-HPLC)

SEC purity was detected by adopting HPLC (Waters e2695 SEC) equipped with an SEC column (TSKgel G3000SWXL, 7.8 mm ID × 30 cm, 5 µm). The mobile phase composition was 1× PBS at a pH of 6.8. The relative percentages of main peaks, aggregates and degradents were calculated by adopting an area normalization method.

### (5) Capillary isoelectric focusing (cIEF)

The cIEF analysis was detected by adopting a capillary electrophoresis apparatus (Beckman, PA800 plus) equipped with a coated capillary (µSIL-FC Capillaries 50 µm ID × 30.2 cm).

### (6) Capillary gel electrophoresis (CE-SDS-R/NR)

CE-SDS-R/NR purity analysis was detected by adopting a capillary electrophoresis apparatus (Beckman, PA800 plus) equipped with an uncoated silica capillary (30.2 cm total length, 50 µm I.D).

### (7) DSC (differential scanning calorimetry)

The thermal stability of the samples was investigated by detecting the thermal transition temperature of the protein with a differential calorimetry scanner (purchased from TA Instruments). The scanning temperature was 10-90 °C, the scanning speed was 1 °C/min, and the obtained profile was integrated by software to obtain the thermal transition temperature midpoint (Tm) value.

### (8) Insoluble particle

A light-blockage method was adopted for detection. An instrument system of an insoluble particle detector (purchased from HIAC) was checked with the filtered and purified water, each sample was measured 4 times, the first measurement data was removed, an average value of three measurement results was taken, and three particle size ranges of 2-10 µm, 10-25 µm and ≥ 25 µm were detected.

### (9) Binding activity

The binding activity experimental method adopted an indirect enzyme-linked immunosorbent assay for detection. Samples were first diluted in a gradient using 1% BSA-PBST diluent at an initial concentration of 10 µg/mL. Then the samples were added into a microplate reader pre-coated with 5 µg/mL antigen CD137-muFc overnight for incubation, and added with detection antibody (1:10000 dilution) labeled by horseradish peroxidase for incubation; the OD value at a wavelength of 450 nm was detected by using a microplate reader after TMB color development. The curve was plotted by a four-parameter fitting regression model based on results, and the relative binding activity of the sample to be tested was calculated by comparing the EC50 value of the sample at day 0 with that of the sample to be tested. The detailed experimental parameters are shown in Table 3.

**Table 3. Experimental parameters for binding activity**

| Experimental steps | Experimental condition | Incubation condition |
|---|---|---|
| Antigen coating | Antigen coating at a concentration of 5 µg/mL, 100 µL per well | 2-8°C, 12-16h |
| Blocking | 3% BSA-PBS blocking solution, 300 µL per well | 37°C, 2h |
| Sample incubation | Sample at an initial concentration of 10 µg/mL, diluted in a 3-fold dilution, with 11 concentrations in total | 37°C, 2h |
| Detection antibody incubation | Detection antibody in 1:10000 dilution, 100 µL per well | 37°C, 1h |
| Color development | After TMB color development with 100 µL added into each well, 50 µL 2N sulfuric acid was added for termination | Room temperature, 10 min |

The performance detection results of preparations 1-14 are shown in Table 4-1, Table 4-2, Table 4-3, Table 4-4, Table 4-5, Table 4-6, Table 4-7, Table 4-8, Table 4-9 and Table 4-10, where "√" represents that the solution is colorless and clear and no impurity is visible, "-" represents undetectable, "D" represents day, "W" represents week, and "T0" represents the starting time point of the investigation.

**Table 4-1. Performance detection results of preparations (appearance)**

| **Prep arati on** | **T0** | **40°C** | | | | | | | |
|---|---|---|---|---|---|---|---|---|---|
| | | **3D** | **10D** | **20D** | | | **30D** | | |
| 1 | √ | √ | √ | Small amount of white fibers, turbid solution | | | Small amount of white fibers, turbid solution | | |
| 2 | √ | √ | √ | Small amount of white fibers, turbid solution | | | Small amount of white fibers, turbid solution | | |
| 3 | √ | √ | √ | Small amount of white fibers, turbid solution | | | Small amount of white fibers, turbid solution | | |

| **Prep arati on** | **T0** | **40°C** | | | **4 °C** | | **Freeze-thaw, -80°C to room temperature** | | |
|---|---|---|---|---|---|---|---|---|---|
| | | **1W** | **2W** | **4W** | **2W** | **4W** | **3 times** | **6 times** | |
| 4 | √ | √ | Slightly turbid solution | Turbid solution | √ | √ | √ | Small amount of white particles | |
| 5 | √ | √ | Slightly turbid solution | Turbid solution | √ | √ | √ | Large number of white particles | |

| **Prep arati on** | **T0** | **25°C** | | | **Shaking** | | **Freeze-thaw, -80 °C to room temperature** | | |
|---|---|---|---|---|---|---|---|---|---|
| | | **1W** | **2W** | **4W** | **1D** | **3D** | **3 times** | | **6 times** |
| 6 | √ | √ | √ | √ | √ | √ | √ | | √ |
| 7 | √ | √ | √ | √ | √ | √ | √ | | √ |

| **Prep arati on** | **T0** | **37°C** | | | | | | | |
|---|---|---|---|---|---|---|---|---|---|
| | | **3W** | | **4W** | | | | | |
| 8 | √ | Slightly turbid solution | | Turbid solution | | | | | |
| 9 | √ | Slightly turbid solution | | Turbid solution | | | | | |
| 10 | √ | Slightly turbid solution | | Turbid solution | | | | | |

| **Prep arati on** | **T0** | **37°C** | | **Freeze-thaw, -80 °C to room temperature** | | | | | |
|---|---|---|---|---|---|---|---|---|---|
| | | **2W** | **3W** | **6 times** | | | | | |
| 11 | √ | Slightly turbid solution | Slightly turbid solution | - | | | | | |
| 12 | √ | Slightly turbid solution | Slightly turbid solution | 4 | | | | | |

| **Prep arati on** | **T0** | **40°C** | | | | | | | |
|---|---|---|---|---|---|---|---|---|---|
| | | **2W** | | **4W** | | | | | |
| 13 | √ | Slightly turbid solution | | Turbid solution | | | | | |
| 14 | √ | Slightly turbid solution | | Turbid solution | | | | | |

It can be seen that preparations 1, 2 and 3 had a small amount of white fibers and a turbid solution after being stored at the high temperature (40 °C) for 30 days. Preparations 4 and 5 had a slightly turbid solution after being investigated at 40 °C for 2 weeks, and had a colorless and clear solution after being investigated at 4 °C for 4 weeks. Preparation 4 had a small amount of white particles after 6 freeze-thaw cycles. Preparations 6 and 7, whether stored at room temperature or shaken or frozen-thawed, all remained a colorless and clear solution. Preparations 8, 9 and 10 had a slightly turbid solution after being investigated at 37 °C for 3 weeks. Preparations 11 and 12 had a slightly turbid solution after being investigated at 37 °C for 3 weeks; preparation 12 had a colorless and clear solution after 6 freeze-thaw cycles. Preparations 13 and 14 had a slightly turbid solution after being investigated at 40 °C for 2 weeks and a turbid solution at 40 °C for 4 weeks.

**Table 4-2. Performance detection results of preparations (pH)**

| **Prep arati on** | **T0** | **40°C** | | | | | | | |
|---|---|---|---|---|---|---|---|---|---|
| | | **3D** | **10D** | **20D** | | | **30D** | | |
| 1 | 6.5 | 6.5 | 6.5 | 6.5 | | | 6.5 | | |
| 2 | 6.6 | 6.5 | 6.5 | 6.5 | | | 6.6 | | |
| 3 | 7.1 | 7.0 | 7.1 | 7.0 | | | 7.1 | | |

| **Prep arati on** | **T0** | **40°C** | | | **4°C** | | **Freeze-thaw, -80 °C to room temperature** | | |
|---|---|---|---|---|---|---|---|---|---|
| | | **1W** | **2W** | **4W** | **2W** | **4W** | **3 times** | **6 times** | |
| 4 | 7.1 | 7.0 | 7.1 | - | 7.1 | 7.1 | 7.0 | 7.1 | |
| 5 | 7.1 | 7.0 | 7.1 | - | 7.1 | 7.1 | 7.0 | 7.1 | |
| **Prep arati on** | **T0** | **25°C** | | | **Shaking** | | **Freeze-thaw, -80 °C to room temperature** | | |
| | | **1W** | \| **2W** | \| **4W** | **1D** | **3D** | **3 times** | | **6 times** |
| 6 | 7.0 | 7.0 | 7.0 | | 7.0 | 7.0 | 7.0 | 7.0 | 7.0 |
| 7 | 7.0 | 7.0 | 7.0 | | 7.0 | 7.0 | 7.0 | 7.0 | 7.0 |

| **Prep arati on** | **T0** | **37°C** | | | | | | | |
|---|---|---|---|---|---|---|---|---|---|
| | | **3W** | | | | | **4W** | | |
| 8 | 7.0 | 7.0 | | | | | 7.0 | | |
| 9 | 7.0 | 7.0 | | | | | 7.0 | | |
| 10 | 7.0 | 7.0 | | | | | 7.1 | | |

| **Prep arati on** | **T0** | **37°C** | | | | | **Freeze-thaw, -80 °C to room temperature** | | |
|---|---|---|---|---|---|---|---|---|---|
| | | **2W** | | **3W** | | | **6 times** | | |
| 11 | 7.0 | 7.0 | | 7.0 | | | - | | |
| 12 | 7.0 | 7.0 | | 7.1 | | | 7.0 | | |

| **Prep arati on** | **T0** | **40°C** | | | | | | | |
|---|---|---|---|---|---|---|---|---|---|
| | | **2W** | | | | | **4W** | | |
| 13 | 6.6 | 6.6 | | | | | 6.6 | | |
| 14 | 7.2 | 7.2 | | | | | 7.2 | | |

It can be seen that none of preparations 1-14 had a significant change in pH.

**Table 4-3. Performance detection results of preparations (protein concentration)**

| **Prep arati on** | **T0** | **40°C** | | | | | | | |
|---|---|---|---|---|---|---|---|---|---|
| | | **3D** | **10D** | **20D** | | | **30D** | | |
| 1 | 9.9 | 10.1 | 10.0 | 9.9 | | | 10.0 | | |
| 2 | 9.9 | 10.1 | 10.0 | 9.9 | | | 10.1 | | |
| 3 | 10.1 | 10.3 | 10.1 | 10.0 | | | 10.5 | | |

| **Prep arati on** | **T0** | **40°C** | | | **4°C** | | **Freeze-thaw, -80 °C to room temperature** | | |
|---|---|---|---|---|---|---|---|---|---|
| | | **1W** | **2W** | **4W** | **2W** | **4W** | **3 times** | **6 times** | |
| 4 | 20.1 | 19.8 | 19.7 | - | 20.1 | 19.3 | 19.3 | 19.3 | |
| 5 | 20.2 | 19.9 | 20.1 | - | 20.2 | 19.7 | 20.2 | 19.7 | |

| **Prep arati on** | **T0** | **25°C** | | | **Shaking** | | **Freeze-thaw, -80 °C to room temperature** | | |
|---|---|---|---|---|---|---|---|---|---|
| | | **1W** | **2W** | **4W** | **1D** | **3D** | **3 times** | | **6 times** |
| 6 | 19.1 | 19.6 | 20.1 | 19.9 | 19.7 | 20.0 | 19.7 | | 19.9 |
| 7 | 18.9 | 19.0 | 19.5 | 19.5 | 19.4 | 18.7 | 19.0 | | 18.8 |

| **Prep arati on** | **T0** | **37°C** | | | | | | | |
|---|---|---|---|---|---|---|---|---|---|
| | | **3W** | | | | **4W** | | | |
| 8 | 20.0 | 20.8 | | | | 19.9 | | | |
| 9 | 19.6 | 20.0 | | | | 19.4 | | | |
| 10 | 19.3 | 20.1 | | | | 19.2 | | | |

| **Prep arati on** | **T0** | **37°C** | | | | **Freeze-thaw, -80 °C to room temperature** | | | |
|---|---|---|---|---|---|---|---|---|---|
| | | **2W** | | **3W** | | **6 times** | | | |
| 11 | 19.5 | 19.9 | | 19.6 | | - | | | |
| 12 | 19.7 | 20.0 | | 20.3 | | 19.4 | | | |

| **Prep arati on** | **T0** | **40 °C** | | | | | | | |
|---|---|---|---|---|---|---|---|---|---|
| | | **2W** | **4W** | | | | | | |
| 13 | 9.8 | 9.7 | 9.9 | | | | | | |
| 14 | 14.2 | 14.3 | 14.9 | | | | | | |

It can be seen that none of preparations 1-14 had a significant change in protein concentration.

**Table 4-4. Performance detection results of preparations (SEC-HPLC)**

| **Prep arati on** | **SEC (%)** | **T0** | **40°C** | | | | | | | |
|---|---|---|---|---|---|---|---|---|---|---|
| | | | **3D** | **10D** | **20D** | | | **30D** | | |
| 1 | Main peak | 98.4 | 96.5 | 94.8 | 91.6 | | | 88.6 | | |
| | Aggreg ate | 1.6 | 3.5 | 5.1 | 7.3 | | | 9.7 | | |
| | Degrad ent | 0.0 | 0.0 | 0.1 | 1.2 | | | 1.7 | | |
| 2 | Main peak | 99.1 | 96.2 | 93.5 | 90.5 | | | 86.1 | | |
| | Aggreg ate | 0.9 | 3.7 | 6.1 | 8.9 | | | 12.5 | | |
| | Degrad ent | 0.0 | 0.2 | 0.4 | 0.6 | | | 1.4 | | |
| 3 | Main peak | 98.2 | 95.1 | 92.4 | 87.6 | | | 81.9 | | |
| | Aggreg ate | 1.8 | 4.7 | 7.0 | 11.1 | | | 15.8 | | |
| | Degrad ent | 0.0 | 0.2 | 0.7 | 1.3 | | | 2.3 | | |

| **Prep arati on** | **SEC (%)** | **T0** | **40°C** | | | **4*C** | | **Freeze-thaw, -80 °C to room temperature** | | |
|---|---|---|---|---|---|---|---|---|---|---|
| | | | **1W** | **2W** | **4W** | **2W** | **4W** | **3 times** | **6 times** | |
| 4 | Main peak | 98.1 | 94.5 | 91.9 | - | 98.0 | 97.6 | 98.0 | 98.1 | |
| | Aggreg ate | 1.9 | 5.0 | 6.8 | - | 2.0 | 2.2 | 2.0 | 2.0 | |
| | Degrad ent | 0.0 | 0.5 | 1.3 | - | 0.0 | 0.2 | 0.0 | 0.0 | |
| 5 | Main peak | 98.1 | 94.4 | 91.7 | - | 98.1 | 97.6 | 98.0 | 98.1 | |
| | Aggreg ate | 2.0 | 5.1 | 7.0 | - | 1.9 | 2.2 | 2.0 | 1.9 | |
| | Degrad ent | 0.0 | 0.5 | 1.3 | - | 0.0 | 0.2 | 0.0 | 0.0 | |

| **Prep arati on** | **SEC (%)** | **T0** | **25°C** | | | **Shaking** | | **Freeze-thaw, -80 °C to room temperature** | | |
|---|---|---|---|---|---|---|---|---|---|---|
| | | | **1W** | **2W** | **4W** | **1D** | **3D** | **3 times** | | **6 times** |
| 6 | Main peak | 99.0 | 98.7 | 98.3 | 97.8 | 98.7 | 98.4 | 98.9 | | 99.0 |
| | Aggreg ate | 1.0 | 1.2 | 1.5 | 1.8 | 1.3 | 1.5 | 1.0 | 1.0 | |
| | Degrad ent | 0.0 | 0.1 | 0.2 | 0.4 | 0.0 | 0.1 | 0.0 | 0.0 | |
| 7 | Main peak | 98.8 | 98.2 | 97.8 | 97.1 | 98.5 | 98.3 | 98.5 | 98.6 | |
| | Aggreg ate | 1.3 | 1.7 | 2.0 | 2.6 | 1.4 | 1.6 | 1.4 | 1.4 | |
| | Degrad ent | 0.0 | 0.2 | 0.3 | 0.4 | 0.1 | 0.1 | 0.1 | 0.0 | |

| **Prep arati on** | **SEC (%)** | **T0** | **37°C** | | | | | | | |
|---|---|---|---|---|---|---|---|---|---|---|
| | | | **3W** | | | | **4W** | | | |
| 8 | Main peak | 99.2 | 92.8 | | | | 91.3 | | | |
| | Aggreg ate | 0.8 | 5.9 | | | | 7.1 | | | |
| | Degrad ent | 0.0 | 1.4 | | | | 1.6 | | | |
| 9 | Main peak | 99.2 | 92.8 | | | | 91.2 | | | |
| | Aggreg ate | 0.8 | 5.9 | | | | 7.3 | | | |
| | Degrad ent | 0.0 | 1.4 | | | | 1.6 | | | |
| 10 | Main peak | 99.2 | 92.7 | | | | 90.8 | | | |
| | Aggreg ate | 0.8 | 6.0 | | | | 7.4 | | | |
| | Degrad ent | 0.0 | 1.3 | | | | 1.7 | | | |

| **Prep arati on** | **SEC (%)** | **T0** | **37°C** | | | | **Freeze-thaw, -80 °C to room temperature** | | | |
|---|---|---|---|---|---|---|---|---|---|---|
| | | | **2W** | | **3W** | | **6 times** | | | |
| 11 | Main peak | 98.9 | 93.8 | | 92.3 | | - | | | |
| | Aggreg ate | 1.0 | 5.6 | | 6.8 | | - | | | |
| | Degrad ent | 0.0 | 0.6 | | 0.9 | | - | | | |
| 12 | Main peak | 99.0 | 93.9 | | 92.6 | | 99.0 | | | |
| | Aggreg ate | 1.0 | 5.4 | | 6.5 | | 1.0 | | | |
| | Degrad ent | 0.0 | 0.7 | | 0.9 | | 0.0 | | | |

| **Prep arati on** | **SEC (%)** | **T0** | **40 °C** | | | | | | | |
|---|---|---|---|---|---|---|---|---|---|---|
| | | | **2W** | | | | **4W** | | | |
| 13 | Main peak | 99.3 | 94.6 | | | | 91.6 | | | |
| | Aggreg ate | 0.6 | 4.7 | 7.4 | | | | | | |
| | Degrad ent | 0.1 | 0.7 | 1.0 | | | | | | |
| 14 | Main peak | 99.3 | 91.9 | 87.0 | | | | | | |
| | Aggreg ate | 0.6 | 6.7 | 11.0 | | | | | | |
| | Degrad ent | 0.0 | 1.3 | 2.0 | | | | | | |

It can be seen that after preparations 1, 2 and 3 were stored at high temperature (40 °C) for 30 days, the increasing speed of the aggregates increased with increasing pH. Preparations 4 and 5 had small main peak purity and low aggregate increasing speed after being investigated at 40 °C for 2 weeks, the main peak purity thereof after 4 weeks of investigation at 4 °C was not significantly different from that at T0, and the main peak content thereof after 6 freeze-thaw cycles was not significantly different. The SEC results of preparations 6 and 7 did not differ much between groups. Preparations 8, 9 and 10 had relatively slowly decreased main peak and less increased aggregate after being investigated at 37 °C for 4 weeks. Preparations 11 and 12 had not large change in SEC main peak purity after being investigated at 37 °C for 3 weeks; preparation 12 had not large change in SEC main peak purity after 6 freeze-thaw cycles. After 4 weeks of investigation at 40 °C for preparations 13 and 14, the data show that the lower the protein concentration, the lower the pH, the higher the sucrose concentration, and the higher the SEC main peak purity.

**Table 4-5. Performance detection results of preparations (cIEF)**

| **Prep arati on** | **cIEF (%)** | **T0** | **40°C** | | | | | | | |
|---|---|---|---|---|---|---|---|---|---|---|
| | | | **3D** | **10D** | **20D** | **30D** | | | | |
| 1 | Main peak | 38.8 | 38.4 | 35.1 | 32.0 | 32.6 | | | | |
| | Acidic peak | 19.4 | 21.6 | 29.1 | 38.8 | 45.7 | | | | |
| | Basic peak | 41.8 | 40.0 | 35.8 | 29.1 | 21.7 | | | | |
| 2 | Main peak | 38.3 | 37.7 | 34.4 | 29.7 | 31.3 | | | | |
| | Acidic peak | 19.2 | 21.9 | 30.4 | 43.6 | 48.6 | | | | |
| | Basic peak | 42.5 | 40.4 | 35.2 | 26.7 | 20.1 | | | | |
| 3 | Main peak | 38.8 | 37.7 | 32.5 | 25.1 | 23.5 | | | | |
| | Acidic peak | 19.7 | 24.3 | 36.8 | 55.1 | 64.3 | | | | |
| | Basic peak | 41.5 | 38.0 | 30.7 | 19.7 | | | 12.2 | | |

| **Prep arati on** | **cIEF (%)** | **T0** | **40°C** | | | **4°C** | | **Freeze-thaw, -80 °C to room temperature** | | |
|---|---|---|---|---|---|---|---|---|---|---|
| | | | **1W** | **2W** | **4W** | **2W** | **4W** | **3 times** | **6 times** | |
| 4 | Main peak | 45.3 | 39.2 | 31.8 | - | 44.3 | 44.9 | 45.3 | 44.1 | |
| | Acidic peak | 20.2 | 33.7 | 46.9 | - | 20.8 | 21.1 | 20.8 | 21.9 | |
| | Basic peak | 34.5 | 27.1 | 21.3 | - | 34.9 | 34.0 | 33.9 | 34.0 | |
| 5 | Main peak | 44.9 | 39.1 | 32.4 | - | 44.6 | 44.7 | 45.4 | 45.2 | |
| | Acidic peak | 20.2 | 32.9 | 46.1 | - | 20.9 | 20.9 | 20.7 | 21.0 | |
| | Basic peak | 34.9 | 28.0 | 21.5 | - | 34.5 | 34.5 | 33.9 | 33.8 | |

| **Prep arati on** | **cIEF (%)** | **T0** | **25°C** | | | **Shaking** | | **Freeze-thaw, -80 °C to room temperature** | | |
|---|---|---|---|---|---|---|---|---|---|---|
| | | | **1W** | **2W** | **4W** | **1D** | **3D** | **3 times** | | **6 times** |
| 6 | Main peak | 55.0 | 53.1 | 51.4 | 48.9 | 54.6 | 53.8 | 53.5 | | 52.1 |
| | Acidic peak | 15.9 | 18.6 | 20.6 | 24.1 | 16.1 | 17.9 | 17.3 | | 18.8 |
| | Basic peak | 29.1 | 28.3 | 28.0 | 26.9 | 29.4 | 28.3 | 29.2 | | 29.1 |
| 7 | Main peak | 54.0 | 53.0 | 51.5 | 47.7 | 54.1 | 53.0 | 53.0 | | 52.5 |
| | Acidic peak | 16.8 | 18.9 | 21.1 | 26.6 | 16.6 | 18.2 | 18.1 | | 18.8 |
| | Basic peak | 29.2 | 28.0 | 27.5 | 25.7 | 29.3 | 28.8 | 28.9 | | 28.7 |

| **Prep arati on** | **cIEF (%)** | **T0** | **37°C** | | | | | | | |
|---|---|---|---|---|---|---|---|---|---|---|
| | | | **3W** | | | | **4W** | | | |
| 8 | Main peak | - | - | | | | - | | | |
| | Acidic peak | - | - | | | | - | | | |
| | Basic peak | - | - | | | | - | | | |
| 9 | Main peak | - | - | | | | - | | | |
| | Acidic peak | - | - | | | | - | | | |
| | Basic peak | - | - | | | | - | | | |
| 10 | Main peak | - | - | | | | - | | | |
| | Acidic peak | - | - | | | | - | | | |
| | Basic peak | - | - | | - | | | | | |

| **Prep arati on** | **cIEF (%)** | **T0** | **37°C** | | **Freeze-thaw, -80 °C to room temperature** | | | | | |
|---|---|---|---|---|---|---|---|---|---|---|
| | | | **2W** | **3W** | **6 times** | | | | | |
| 11 | Main peak | - | - | - | - | | | | | |
| | Acidic peak | - | - | - | - | | | | | |
| | Basic peak | - | - | - | - | | | | | |
| 12 | Main peak | - | - | - | - | | | | | |
| | Acidic peak | - | - | - | - | | | | | |
| | Basic peak | - | - | - | - | | | | | |

| **Prep arati on** | **cIEF (%)** | **T0** | **40°C** | | | | | | | |
|---|---|---|---|---|---|---|---|---|---|---|
| | | | **2W** | | **4W** | | | | | |
| 13 | Main peak | 58.2 | - | | 32.4 | | | | | |
| | Acidic peak | 15.3 | - | | 54.2 | | | | | |
| | Basic peak | 26.6 | - | | 13.4 | | | | | |
| 14 | Main peak | 58.4 | - | | 23.5 | | | | | |
| | Acidic peak | 15.6 | - | | 70.6 | | | | | |
| | Basic peak | 25.9 | - | | 5.9 | | | | | |

It can be seen that after preparations 1, 2 and 3 were stored at high temperature (40 °C) for 30 days, they all had significantly decreased main peak and basic peak and significantly increased acidic peak. Preparations 4 and 5 all had significantly decreased main peak and basic peak and significantly increased acidic peak after being investigated at 40 °C for 2 weeks, the main peak purity thereof after 4 weeks of investigation at 4 °C was not significantly different from that at T0, and the main peak purity thereof after 6 freeze-thaw cycles was not significantly different from that at T0. The cIEF results of preparations 6 and 7 did not differ much between groups; preparations 13 and 14 had significantly decreased main peak and significantly increased acidic peak and basic peak after being investigated at 40 °C for 4 weeks.

**Table 4-6. Performance detection results of preparations (CE-SDS-R)**

| **Preparati on** | **CE-SDS-R (%)** | **T0** | **3D** | **10D** | **40 °C** | | | | | |
|---|---|---|---|---|---|---|---|---|---|---|
| | | | | | **20D** | | | **30D** | | |
| 1 | Main peak | 97.5 | 96.7 | 94.7 | 92.4 | | | 91.9 | | |
| 2 | Main peak | 97.4 | 95.8 | 94.3 | 90.8 | | | 88.5 | | |
| 3 | Main peak | 97.7 | 95.5 | 92.6 | 87.8 | | | 83.6 | | |

| **Prep arati on** | **CE-SD S-R (%)** | **T0** | **40°C** | | | **4°C** | | **Freeze-thaw, -80 °C to room temperature** | | |
|---|---|---|---|---|---|---|---|---|---|---|
| | | | **1W** | **2W** | **4W** | **2W** | **4W** | **3 times** | **6 times** | |
| 4 | Main peak | 97.4 | 93.3 | 89.6 | - | 97.1 | 97.0 | 97.3 | 97.1 | |
| 5 | Main peak | 97.2 | 93.8 | 89.9 | - | 97.2 | 97.0 | 97.2 | 96.8 | |

| **Prep arati on** | **CE-SD S-R (%)** | **T0** | **25°C** | | | **Shaking** | | **Freeze-thaw, -80 °C to room temperature** | | |
|---|---|---|---|---|---|---|---|---|---|---|
| | | | **1W** | **2W** | **4W** | **1D** | **3D** | **3 times** | | **6 times** |
| 6 | Main peak | 97.8 | 97.7 | 97.1 | 96.5 | 97.8 | 97.7 | 98.2 | | 98.1 |
| 7 | Main peak | 97.9 | 97.6 | 97.0 | 96.0 | 97.8 | 97.7 | 98.2 | | 97.9 |
| **Prep arati on** | **CE-SD S-R (%)** | **T0** | **37°C** | | | | | | | |
| | | | **3W** | | | | **4W** | | | |
| 8 | Main peak | 97.7 | 91.8 | | | | 89.6 | | | |
| 9 | Main peak | 97.8 | 91.9 | | | | 89.7 | | | |
| 10 | Main peak | 97.8 | 91.9 | | | | 89.4 | | | |

| **Prep arati on** | **CE-SD S-R (%)** | **T0** | **37°C** | | | | **Freeze-thaw, -80 °C to room temperature** | | | |
|---|---|---|---|---|---|---|---|---|---|---|
| | | | **2W** | | **3W** | | **6 times** | | | |
| 11 | Main peak | 97.5 | 94.0 | | 91.3 | | - | | | |
| 12 | Main peak | 97.6 | 93.8 | | 91.4 | | 97.8 | | | |

| **Prep arati on** | **CE-SD S-R (%)** | **T0** | **40 °C** | | | | | | | |
|---|---|---|---|---|---|---|---|---|---|---|
| | | | **2W** | | | | **4W** | | | |
| 13 | Main peak | 98.5 | 94.6 | | | | 90.6 | | | |
| 14 | Main peak | 98.5 | 91.9 | | | | 85.4 | | | |

It can be seen that the CE-SDS-R main peak purity of preparations 1, 2 and 3 after being stored at the high temperature (40 °C) for 30 days decreased by 5.5%, 8.9% and 14.1%, respectively. Preparations 4 and 5 all had significantly decreased main peak purity after being investigated at 40 °C for 2 weeks, the main peak purity thereof after 4°C of investigation at 4 °C was not significantly different from that at T0, and the main peak purity thereof after 6 freeze-thaw cycles was not significantly different from that at T0. The CE-SDS-R results of preparations 6 and 7 did not differ much between groups. Preparations 8, 9 and 10 had less decreased CE-SDS-R main peak purity after being investigated at 37 °C for 4 weeks. The main peak of preparations 11 and 12 after being investigated at 37 °C for 3 weeks decreased by 6.2% and 6.3%, respectively; preparation 12 had not large change in CE-SDS-R main peak purity after 6 freeze-thaw cycles. Preparations 13 and 14 had not large change in CE-SDS-R main peak purity after being investigated at 40 °C for 4 weeks.

**Table 4-7, Performance detection results of preparations (CE-SDS-NR)**

| **Prep arati on** | **CE-SDS -NR (%)** | **T0** | **40°C** | | | | | | | |
|---|---|---|---|---|---|---|---|---|---|---|
| | | | **3D** | **10D** | **20D** | | | **30D** | | |
| 1 | Main peak | 97.2 | 96.6 | 94.7 | 90.1 | | | 87.2 | | |
| 2 | Main peak | 97.2 | 96.2 | 94.0 | 88.4 | | | 85.2 | | |
| 3 | Main peak | 97.3 | 95.8 | 91.4 | 83.0 | | | 79.2 | | |

| **Prep arati on** | **CE-SDS -NR (%)** | **T0** | **40°C** | | | **4°C** | | **Freeze-thaw, -80 °C to room temperature** | | |
|---|---|---|---|---|---|---|---|---|---|---|
| | | | **1W** | **2W** | **4W** | **2W** | **4W** | **3 times** | **6 times** | |
| 4 | Main peak | 96.5 | 91.4 | 86.8 | - | 96.1 | 96.4 | 96.2 | 95.9 | |
| 5 | Main peak | 96.4 | 91.5 | 86.9 | - | 96.3 | 96.1 | 96.0 | 96.0 | |

| **Prep arati on** | **CE-SDS -NR (%)** | **T0** | **25°C** | | | **Shaking** | | **Freeze-thaw, -80 °C to room temperature** | | |
|---|---|---|---|---|---|---|---|---|---|---|
| | | | **1W** | **2W** | **4W** | **1D** | **3D** | **3 times** | | **6 times** |
| 6 | Main peak | 98.5 | 98.1 | 97.4 | 96.5 | 98.3 | 98.0 | 98.4 | | 97.9 |
| 7 | Main peak | 98.5 | 98.0 | 97.1 | 96.2 | 98.3 | 98.2 | 97.9 | | 98.0 |

| **Prep arati on** | **CE-SDS -NR (%)** | **T0** | **37°C** | | | | | | | |
|---|---|---|---|---|---|---|---|---|---|---|
| | | | **3W** | | | | **4W** | | | |
| 8 | Main peak | 98.4 | 88.0 | | | | 86.0 | | | |
| 9 | Main peak | 98.4 | 88.0 | | | | 85.8 | | | |
| 10 | Main peak | 98.4 | 87.7 | | | | 85.7 | | | |

| **Prep aration** | **CE-SDS -NR (%)** | **T0** | **37°C** | | **Freeze-thaw, -80 °C to room temperature6 times** | | | | | |
|---|---|---|---|---|---|---|---|---|---|---|
| | | | **2W** | **3W** | | | | | | |
| 11 | Main peak | 98.0 | 91.6 | 90.0 | - | | | | | |
| 12 | Main peak | 98.1 | 91.3 | 89.6 | 98.0 | | | | | |

| **Prep arati on** | **CE-SDS -NR (%)** | **T0** | **40°C** | | | | | | | |
|---|---|---|---|---|---|---|---|---|---|---|
| | | | **2W** | | **4W** | | | | | |
| 13 | Main peak | 98.7 | 92.3 | | 88.0 | | | | | |
| 14 | Main peak | 98.6 | 88.0 | | 79.1 | | | | | |

It can be seen that the CE-SDS-NR main peak purity of preparations 1, 2 and 3 after being stored at the high temperature (40 °C) for 30 days decreased by 10.1%, 12.1% and 18.1%, respectively. Preparations 4 and 5 all had significantly decreased main peak purity after being investigated at 40 °C for 2 weeks, the main peak purity thereof after 4°C of investigation at 4 °C was not significantly different from that at T0, and the main peak purity thereof after 6 freeze-thaw cycles was not significantly different from that at T0. The CE-SDS-NR results of preparations 6 and 7 did not differ much between groups. Preparations 8, 9 and 10 had less decreased CE-SDS-NR main peak purity after being investigated at 37 °C for 4 weeks. The main peak of preparations 11 and 12 after being investigated at 37 °C for 3 weeks decreased by 8.1% and 8.3%, respectively; preparation 12 had not large change in CE-SDS-NR main peak purity after 6 freeze-thaw cycles. Preparations 13 and 14 had not large change in CE-SDS-NR main peak purity after being investigated at 40 °C for 4 weeks.

**Table 4-8. Performance detection results of preparations (DSC)**

| **Prep arati on** | **Tm1** | **Tm2** |
|---|---|---|
| 4 | 60.2 | 71.7 |
| 5 | 60.2 | 71.8 |

It can be seen that preparations 4 and 5 had higher Tm value and relatively better thermal stability.

**Table 4-9. Performance detection results of preparations (insoluble particles)**

| **Preparation** | **T0** | | | **1D** | | | **3D** | | |
|---|---|---|---|---|---|---|---|---|---|
| | **2µm** | **10µm** | **25µm** | **2µm** | **10µm** | **25µm** | **2µm** | **10µm** | **25µm** |
| 6 | 284.67 | 18.67 | 0.00 | 378.67 | 28.00 | 0.33 | 1372.00 | 40.33 | 1.00 |
| 7 | 200.00 | 19.67 | 4.33 | 1992.33 | 126.67 | 1.67 | 350.33 | 12.33 | 0.00 |

| **Preparation** | **T0** | | | **Freeze-thaw, -80 °C to room temperature, 6 cycles** | | | | | |
|---|---|---|---|---|---|---|---|---|---|
| | **2µm** | **10µm** | **25µm** | **2µm** | | **10µm** | | **25µm** | |
| 12 | 556.00 | 13.33 | 0.33 | 1629.00 | | 79.33 | | 1.00 | |

It can be seen that preparations 6 and 7 had fewer insoluble particles; preparation 12 had fewer insoluble particles.

**Table 4-10. Performance detection results of preparations (binding activity)**

| **Preparation** | **EC50 (ng/ml)** | | **Binding activity** | |
|---|---|---|---|---|
| | **T0** | **4W** | **T0** | **4W** |
| 13 | 17.14 | 22.87 | 100% | 74.9% |
| 14 | 19.44 | 24.32 | 100% | 79.9% |

It can be seen that preparations 13 and 14 had relatively better binding activity.

### Example 3. Therapeutic Effect of Preparation on Tumors In Vivo

Construction of CT26 tumor model mice:
(1) Mouse colon cancer cells CT26 cells (purchased from ATCC) were recovered, and digested 5 days after recovery; the digested cells were collected. The digested cells were washed with PBS, filtered through a strainer, counted, and resuspended to obtain cell suspension at a concentration of 5 × 10⁶ cells/mL, where CT26 cells had a cell viability of about 94%.
(2) 40 Balbc-KI mice (purchased from GemPharmatech Co., Ltd.) were each inoculated subcutaneously with 100 µL of cell suspension obtained in step (1). After inoculation, the mice were observed to be tumor-bearing, thereby obtaining CT26 tumor model mice.

Administration of preparation described herein for the treatment of tumors:
(1) Preparation 13 prepared in Example 2 and a solution containing human IgG4 in the control group (where all other components and contents in the solution were the same as those in preparation 13 except that the solute was human IgG4 instead of CD137 antibody) were administered to the CT26 tumor model mice according to Table 5.

**Table 5**

| Group | Administered preparation | Administered dose (mg/Kg) | Preparation concentration | Administration mode | Administration cycle |
|---|---|---|---|---|---|
| 1 | Solution comprising human IgG4 | 5 | 5.7mg/mL | Intraperitoneal | 2 times weekly for 4 weeks |
| 2 | Preparation 13 | 1 | 10 mg/mL | Intraperitoneal | 2 times weekly for 4 weeks |
| 3 | Preparation 13 | 5 | 10 mg/mL | Intraperitoneal | 2 times weekly for 4 weeks |

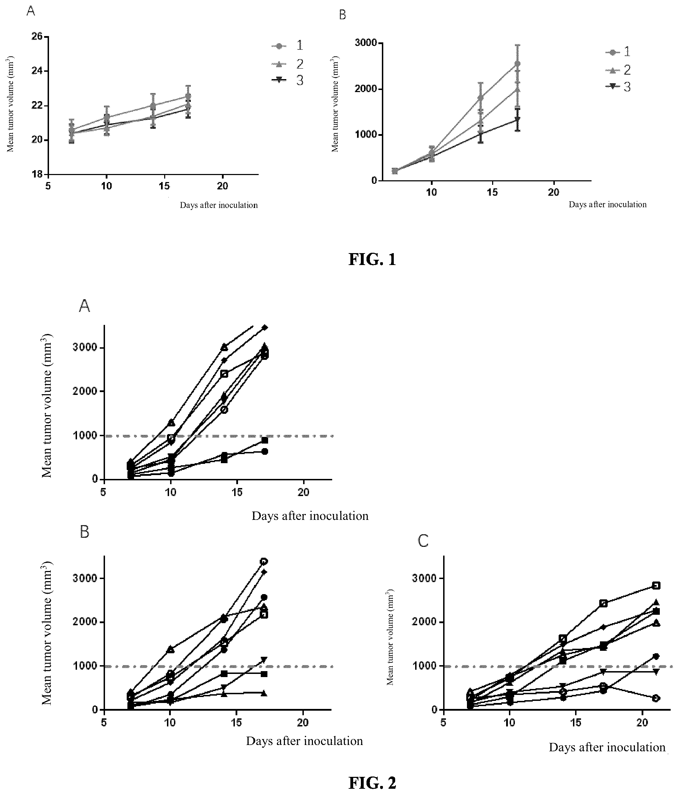

The situations after administration are shown in FIGs. 1A-1B, where 1-3 are the situations of groups 1-3 in sequence. FIG. 1A and FIG. 1B show the results of two repeated experiments, respectively. The results in FIG. 1 show that preparation xx prepared in Example 2 can significantly improve the killing effect on mouse colon cancer cells relative to the control group, and the larger the administered dose, the more significant the killing effect.

FIGs. 2A-2C also reflect the killing effect of groups 1-3 on mouse colon cancer cells in sequence, which reflects *in vivo* tumor volume of each mouse in each group. The results in FIG. 2 show that preparation xx prepared in Example 2 significantly improves the killing effect on mouse colon cancer cells relative to the control group, and the larger the administered dose, the more significant the killing effect.

### Example 4. Enhanced Secretion of Cytokines by Preparation

(1) 0.1 µg/mL of CD3 antibody (from Biolegend 317304) was plated and let stand overnight at 4 °C.
(2) The plate was washed 2 times with PBS solution, and preparation 13 at different concentrations prepared in Example 2 was plated and allowed to stand at 37 °C for 2 h.
(3) The plate was washed 2 times with PBS solution, human T cells at a concentration of 200000 cells/well were separated from fresh blood of a healthy person, and the cells were added into the plate treated in the above step (2) and reacted at 37 °C for 3 h.
(4) The supernatant was collected, and the content of human IFN-g in the supernatant was detected.

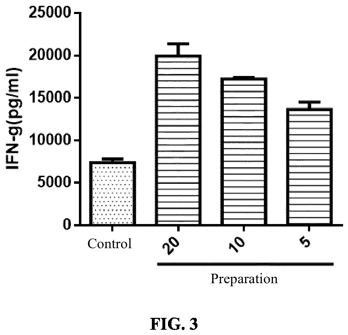

The results are shown in FIG. 3, where the control was a solution containing human IgG4 in Example 3 and the preparation was preparation 13 prepared in Example 2. The results in FIG. 3 show that preparation 13 prepared in Example 2 can significantly improve the ability of T cells to secrete IFN-g relative to the control group, which is favorable for improving the ability to kill tumors.

The foregoing detailed description is provided by way of explanation and examples, and is not intended to limit the scope of the appended claims. Various changes of the embodiments listed in the present application until now would be obvious to those of ordinary skills in the art, and should be kept within the scope of the appended claims and equivalents thereof.

## Claims

1. A preparation, comprising an antibody or an antigen-binding fragment thereof that specifically binds to CD137, wherein the CD137 antibody comprises a light chain variable region VL and a heavy chain variable region VH, wherein the VL comprises an amino acid sequence as set forth in any one of SEQ ID NOs: 1-11; and the VH comprises an amino acid sequence as set forth in any one of SEQ ID NOs: 12-22.

2. The preparation according to claim 1, wherein
1) the VL comprises an amino acid sequence as set forth in SEQ ID NO: 1, and the VH comprises an amino acid sequence as set forth in SEQ ID NO: 12;
2) the VL comprises an amino acid sequence as set forth in SEQ ID NO: 3, and the VH comprises an amino acid sequence as set forth in SEQ ID NO: 14;
3) the VL comprises an amino acid sequence as set forth in SEQ ID NO: 5, and the VH comprises an amino acid sequence as set forth in SEQ ID NO: 16;
4) the VL comprises an amino acid sequence as set forth in SEQ ID NO: 7, and the VH comprises an amino acid sequence as set forth in SEQ ID NO: 18;
5) the VL comprises an amino acid sequence as set forth in SEQ ID NO: 8, and the VH comprises an amino acid sequence as set forth in SEQ ID NO: 19;
6) the VL comprises an amino acid sequence as set forth in SEQ ID NO: 9, and the VH comprises an amino acid sequence as set forth in SEQ ID NO: 20;
7) the VL comprises an amino acid sequence as set forth in SEQ ID NO: 10, and the VH comprises an amino acid sequence as set forth in SEQ ID NO: 21;
8) the VL comprises an amino acid sequence as set forth in SEQ ID NO: 11, and the VH comprises an amino acid sequence as set forth in SEQ ID NO: 22.

3. The preparation according to any one of claims 1-2, wherein the preparation comprises a buffer salt selected from one or more of the group consisting of: phosphate, citrate and acetate.

4. The preparation according to any one of claims 1-3, wherein the preparation comprises a buffer salt comprising disodium hydrogen phosphate, sodium dihydrogen phosphate and/or sodium citrate.

5. The preparation according to any one of claims 1-4, wherein the preparation comprises a buffer salt at a concentration of 5-50 mM.

6. The preparation according to any one of claims 1-5, wherein the preparation comprises a stabilizer selected from one or more of the group consisting of: sucrose, trehalose, mannitol, glycine, arginine hydrochloride, sodium citrate, histidine, sodium acetate and sodium chloride.

7. The preparation according to any one of claims 1-6, wherein the preparation comprises a stabilizer comprising sucrose.

8. The preparation according to any one of claims 1-7, wherein the preparation comprises a stabilizer comprising sucrose and sodium citrate.

9. The preparation according to any one of claims 1-8, wherein the preparation comprises a stabilizer at a concentration of 1%-10% (wt).

10. The preparation according to any one of claims 1-9, wherein the preparation comprises a surfactant selected from one or more of the group consisting of: polysorbate 20 and polysorbate 80.

11. The preparation according to any one of claims 1-10, wherein the preparation comprises a surfactant at a concentration of 0.01%-0.2% (wt).

12. The preparation according to any one of claims 1-11, wherein the preparation has a pH of 6.5-7.5.

13. The preparation according to any one of claims 1-12, wherein the preparation comprises:
sucrose, polysorbate 80, disodium hydrogen phosphate, sodium dihydrogen phosphate, sodium citrate and water.

14. A kit, comprising the preparation according to any one of claims 1-13 and a container for containing the preparation.

15. Use of the preparation according to any one of claims 1-13 or the kit according to claim 14 in the preparation of a drug for treating cancer.

16. The use according to claim 15, wherein the cancer is selected from the group consisting of: melanoma, prostate cancer, colorectal cancer, Merkel cell carcinoma, pancreatic cancer, non-Hodgkin's lymphoma, squamous cell carcinoma and breast cancer.
